# FASCICULE DE BREVET EUROPEEN

(11) **EP 0 617 737 B1**
(45) Date de publication et mention de la délivrance du brevet: **15.01.1997**
(21) Numéro de dépôt: 92923617.2
(22) Date de dépôt: 30.11.1992
(51) Int. Cl.: C12N 15/52, C12N 9/00, C12N 11/06, C07K 17/06, C12Q 1/25

(54) **PROCEDE DE SELECTION DE MICROORGANISMES RECOMBINANTS COMPORTANT A LEUR SURFACE AU MOINS UNE MOLECULE A ACTIVITE ENZYMATIQUE**
Verfahren zur Selektionrekombinanter Mikroorganismen die auf ihre Oberfläche wenigstens ein Molekül mit enzymatischer Aktivität tragen.
METHOD FOR SELECTING RECOMBINANT MICRO-ORGANISMS OF WHICH THE SURFACE COMPRISES AT LEAST ONE MOLECULE HAVING ENZYMATIC ACTIVITY

(30) Priorité: 29.11.1991 BE 9101106
(43) Date de publication de la demande: 05.10.1994
(73) Titulaire: UNIVERSITE CATHOLIQUE DE LOUVAIN, B-1348 Louvain-la-Neuve (BE)
(72) Inventeur: FASTREZ, Jacques, B-1360 Thorembais-S.-Trond (BE)
(74) Mandataire: Van Malderen, Michel
(86) Numéro de dépôt international: BE9200052
(87) Numéro de publication internationale: WO9311242

(56) Documents cités:
- EP-A- 0 423 938
- WO-A-88/04326
- WO-A-90/04041
- DE-A- 3 833 628
- FR-A- 2 210 620
- US-A- 4 165 258
- SCIENCE, vol. 249, 27 juillet 1990, Lancaster, PA (US); J.J. DEVLIN et al., pp. 404-406
- PATENT ABSTRACTS OF JAPAN, vol. 015, no. 248 (C-843), 25 juin 1991
- J.M. FRERE et al., "Les méthodes de purification et d'analyses des protéines", 1981, Masson, Paris (FR)
- PATENT ABSTRACTS OF JAPAN, vol. 13, no. 171 (C-588), 24 april 1989

## Description

### Objet de l'invention

L'invention concerne un procédé de sélection de microorganismes recombinants comportant à leur surface au moins une molécule à activité enzymatique.

L'invention s'étend également aux moyens pour la mise en oeuvre de ce procédé et à leurs procédés d'obtention.

### Etat de la technique et arrière-plan technologique à la base de l'invention

La découverte d'enzymes ayant des propriétés nouvelles (activité catalytique, nouvelle stabilité accrue, spécificité altérée, profil pH déplacé,...) est d'un grand intérêt pour l'amélioration des processus biochimiques.

Quoique des progrès très substantiels aient été réalisés dans la compréhension de la relation structure-activité des protéines (Atkins et al., Current opinion in structural biology (1991), 1, p. 611 à 623), la prédiction du type de modification à introduire dans une protéine pour altérer ses propriétés dans une direction recherchée reste difficile.

En conséquence, on recourt habituellement à une stratégie dans laquelle on part d'une enzyme existante dont on mute le gène. On réalise cette mutagenèse au hasard, en créant ainsi une vaste population d'enzymes mutées. Parmi celles-ci, on espère que certaines auront les propriétés nouvelles recherchées (Cunningham et al., Protein Engineering (1987), 1, p. 319 à 325 et Lehtovaara et al. Protein Engineering (1988), 2, p. 263 à 268).

Pour augmenter les chances de succès, il est nécessaire de tester un très grand nombre de mutants et de disposer d'une technique qui permette de sélectionner, dans la population la plus grande possible, les enzymes ayant les propriétés adéquates.

Des protéines possédant des propriétés enzymatiques nouvelles peuvent aussi être produites en recourant à la technique des anticorps monoclonaux. Dans ce cas, la sélection des anticorps-enzymes (appelés aussi abzymes) présentant les propriétés adéquates nécessite également une technique de criblage efficace.

Une technique de détection des enzymes mutées fréquemment utilisée repose sur la mise au point d'un test sensible pour l'activité enzymatique. On cherche alors directement sur une population de mutants étalés sur boîte de Pétri ceux qui produisent l'enzyme souhaitée. Cependant, cette technique est laborieuse et ne permet de tester qu'un nombre limité de clones (Pollack et al., J. Am. Chem. Soc., 111, p. 5961-5962 (1989) et Huse et al., Science, 246, p. 1275-1281 (1989)).

Une alternative avantageuse se présente lorsque l'activité catalytique recherchée peut être profitable à la survie ou au développement du micro-organisme exprimant le gène correspondant. Il suffit alors d'exercer une pression de sélection sur le milieu de culture de la population de mutants pour sélectionner les souches qui produisent l'enzyme souhaitée (Hermès et al., PNAS, USA, 87, p. 696-700 (1990) et Evnin et al., PNAS, USA, 87, p. 6659-6663 (1990)).

Cette technique présente le désavantage de ne pouvoir être appliquée à toutes les molécules à activité enzymatique.

Il est également connu (Smith, Science (1985), 228, p. 1315-1317, Parmley et Smith, Gene (1985), 76, 305-318, de la Cruz et al., J. Biol. Chem. (1988), 263, p. 4318-4322, Bass et al., Proteins (1990), 8, p. 309 à 314, Cwirla et al., Proc. Natl. Acad. Sci. USA (1990), 87, p. 6378-6382, Devlin et al., Science (1990), 249, 404-406, McCafferty et al., Nature (1990), 348, p. 552-554, Scott et Smith, Science (1990), 249, p. 386-390, Clackson et al., Nature (1991), 352, p. 624-628, Lowman et al., Biochemistry, (1991), 30, p. 10832-10838, J. McCafferty et al., Prot. Engng (1991) 955-961, Kang et al., (1991) Proc. Natl. Acad. Sci. USA, vol. 88, p. 4363-4366; Barbas et al. (1991), Proc. Natl. Acad. Sci. USA, vol. 88, p. 7978-7982; Roberts et al., Proc. Natl. Acad. Sci. USA (1992), 89, p. 2429-2433) de pouvoir exposer certaines séquences peptidiques étrangères, en particulier des protéines, à la surface de phages "filamenteux" et de phagemides.

Cependant, la sélection in vitro des phages exposant correctement à leur surface des séquences polypeptidiques actives n'est pas non plus résolue.

Diverses techniques de chromatographie d'affinité ont été décrites. Parmi les supports d'affinité mis au point, plusieurs utilisent de la streptavidine ou de l'avidine immobilisée, celle-ci se trouvant avantageusement sous forme monomérique (EP-A-0 423 938). Des phages présentant des séquences peptidiques ayant une affinité pour la streptavidine ont ainsi été sélectionnés à l'aide d'agarose porteur de cette protéine (Devlin et al., Science (1990), 249, 404-406).

Cependant, ces techniques présentent l'inconvénient que la sélection en chromatographie d'affinité de ces phages s'effectue sur base de leur faculté à se complexer avec des récepteurs peptidiques ou des anticorps spécifiques qui fixent indifféremment les molécules actives ou non actives.

Dans le but de détecter des microorganismes particuliers, diverses autres techniques de marquage ont été élaborées. Par exemple, certains microorganismes ont été détectés à l'aide de bactériophages spécifiques pour ces hôtes et marqués (WO-A-8 804 326). Des particules transductrices de spécificité d'hôte connues et contenant des gènes altérant le phénotype peuvent être utilisées pour le marquage de bactéries (WO-A-9 004 041).

La sensibilité à certaines substances toxiques associée à la détection par bioluminescence encodée par un plasmide a aussi été utilisée pour détecter des microorganismes particuliers.

Cependant aucune de ces techniques ne permet de sélectionner des microorganismes porteurs de peptides catalytiques.

D'autre part, des inhibiteurs covalents, utilisés comme substrat par certaines enzymes, sont également connus pour leur capacité à inhiber toute réaction enzymatique en se fixant de manière covalente, au site actif de l'enzyme.

Certains inhibiteurs covalents sont également dénommés inhibiteurs "suicides" (ATOR et al., The Enzyme, vol. 19, p. 214-282, edit. Sigman Boyer 3th edition (1990)) car ils se fixent de manière irréversible au site actif de l'enzyme en utilisant le mécanisme catalytique de celle-ci et rendent ainsi toute réutilisation de l'enzyme impossible.

Il est également connu qu'il est possible d'immobiliser un substrat en liant d'abord celui-ci à de la biotine et en mettant le produit obtenu en présence d'avidine liée à un support insoluble dans l'eau (JP-A-3 080 098). Ce substrat immobilisé permet la détermination aisée de certaines activités enzymatiques, mais ne peut servir de support chromatographique pour sélectionner des microorganismes porteurs de peptides catalytiques.

### Buts de l'invention

L'invention vise à mettre au point un procédé de sélection de microorganismes recombinants comportant à leur surface au moins une molécule à activité enzymatique et qui ne comporte pas les inconvénients de l'état de la technique.

L'invention vise en particulier à mettre au point un procédé qui permette la détection et la sélection des microorganismes recombinants exposant à leur surface des molécules à activité enzymatique, qui s'effectuent sur base de l'activité catalytique de ces molécules plutôt que sur base de leur activité de complexation.

### Eléments caractéristiques de l'invention

L'invention concerne un procédé de sélection de microorganismes recombinants comportant à leur surface au moins une molécule à activité enzymatique, dans lequel les microorganismes recombinants, de préférence des virus tels que des phages filamenteux ou des phages λ ou des phagemides, sont immobilisés sur un support solide par un inhibiteur covalent du site actif de la molécule à activité enzymatique, relié par un bras au support solide; les microorganismes recombinants ou tout ou une partie de leur génome sont ensuite recueillis et tout ou une partie de leur génome est isolé.

On entend par le terme "microorganisme recombinant" toute structure macromoléculaire satisfaisant à la définition anglaise de "Genetic Display Package" (GDP), c'est-à-dire toute structure macromoléculaire incorporant du matériel génétique et comportant une séquence peptidique codée par ce matériel génétique.

Cette définition inclut notamment les levures, les bactéries, les virus, les phages tels que les phages filamenteux ou les phagemides.

On entend par le terme "support solide" toute structure solide sur laquelle les microorganismes peuvent être provisoirement immobilisés.

Un tel support solide peut être réalisé par l'homme de l'art suivant les techniques connues de l'état de la technique.

Ce support peut être constitué par un support chromatographique, des particules paramagnétiques, un support de polystyrène ou tout autre support sur lesquels sont immobilisées des protéines complexantes telles que de l'avidine ou de la streptavidine ou un anticorps capables de fixer le microorganisme.

Préférentiellement, les molécule à activité enzymatique sont choisies parmi le groupe constitué par les enzymes, les abzymes, les peptides catalytiques ou un mélange d'entre eux.

Selon une forme d'exécution préférée du procédé de l'invention, préalablement à leur immobilisation sur le support solide, les microorganismes recombinants sont marqués par l'inhibiteur covalent relié par un bras à un ligand immobilisable sur le support solide.

Selon une autre forme d'exécution préférée du procédé de l'invention, les microorganismes ou tout ou une partie de leur génome sont recueillis par destruction du microorganisme ou par détachement du microorganisme de son support solide.

Ce détachement peut être effectué selon plusieurs procédés: soit en détachant le ligand du support solide avec une solution éluante, de préférence avec une solution à un pH acide; soit en clivant le bras reliant l'inhibiteur covalent au support solide; soit en clivant la liaison entre l'inhibiteur covalent et la molécule à activité enzymatique; soit en clivant un lien peptidique entre la molécule à activité enzymatique et le microorganisme.

Le clivage du lien peptidique entre la molécule à activité enzymatique s'effectue de préférence par un protéase spécifique tel que le facteur 10A ou la collagénase; le bromure de cyanogène clivant un lien peptidique comportant une méthionine ou l'hydroxylamine suivant un lien peptidique comportant le peptide asparagine-glycine peuvent être également utilisées.

Un tel clivage permet d'augmenter avantageusement la sélectivité du procédé selon l'invention.

En effet, l'inhibiteur covalent dans le procédé selon l'invention est susceptible de se fixer également sur le microorganisme sur d'autres sites que le site actif de la molécule à activité enzymatique.

En clivant le lien peptidique spécifique existant entre la molécule à activité enzymatique et le micro-organisme, seul le microorganisme comportant à sa surface une molécule enzymatique active sera recueilli lors dé l'élution.

De plus, ce procédé de sélection peut être appliqué pour la détection et la sélection de microorganismes exposant à leur surface sur une protéine native ou recombinante du microorganisme, un ou plusieurs peptides particuliers susceptibles d'induire une réponse immunitaire cellulaire ou humorale.

En effet, il a été constaté qu'une simple reconnaissance anticorps antigène de tels peptides ne permettait pas de sélectionner les peptides les plus aptes à susciter une réponse immunitaire cellulaire ou humorale (Wetzel (1991), Prot. Eng., vol 4, p. 371-374).

Par conséquent, un clivage réalisé selon l'invention au niveau du lien peptidique existant entre ledit peptide et la molécule native du microorganisme permet également de sélectionner de manière spécifique le ou les peptides les plus aptes à susciter une réaction cellulaire ou humorale.

En outre, tout ou une partie du génome des microorganismes peut être également recueillie en détruisant le microorganisme et en récupérant tout ou une partie de son génome. Ce génome est ensuite isolé, amplifié (par exemple par PCR) et cloné.

L'invention concerne également un marqueur constitué par un inhibiteur covalent, de préférence irréversible, du site actif d'une molécule à activité enzymatique, relié par un bras à un ligand immobilisable sur un support solide.

De préférence, le ligand est une biotine ou une imino-biotine.

Avantageusement, le bras comporte une fonction chimique clivable, de préférence une fonction disulfure.

Selon une forme d'exécution préférée du marqueur selon l'invention, l'inhibiteur covalent est un analogue de l'état de transition spécifique de la réaction enzymatique réalisé par la molécule à activité enzymatique.

Un autre aspect de l'invention concerne un procédé de préparation du marqueur selon l'invention, dans lequel on couple par un bras un inhibiteur covalent du site actif d'une molécule à activité enzymatique à un ligand immobilisable sur un support solide.

L'invention concerne aussi un support solide dans lequel un inhibiteur covalent du site actif d'une molécule à activité enzymatique est relié par un bras à un support solide.

Avantageusement, l'inhibiteur covalent relié au support solide est constitué par le marqueur selon l'invention.

Selon une forme d'exécution préférée de l'invention, le bras est relié au support solide par un lien ester ou comporte un diol vicinal.

Un dernier aspect de l'invention concerne le procédé de préparation du support solide selon l'invention dans lequel on relie par un bras, un inhibiteur covalent du site actif d'une molécule à activité enzymatique à un support solide.

### Brève description des figures

- - La figure 1: représente la séquence nucléotidique du gène codant pour la β-lactamase du plasmide pBR322.
- - La figure 2: représente de manière schématique les étapes du procédé d'insertion du gène codant pour la β-lactamase mature, dans le phage fdDOG1.
- - La figure 3: représente les séquences nucléotidiques des amorces complémentaires du gène codant pour la β-lactamase.
- - Les figures 4 à 9: représentent de manière schématique le procédé de synthèse des marqueurs selon l'invention.
- - La figure 10: représente de manière schématique la fixation d'un microorganisme sur un support solide, par le marqueur selon l'invention, ainsi que les sites de clivage possibles permettant de recueillir ledit microorganisme.

### Description d'un mode d'exécution préféré de l'invention

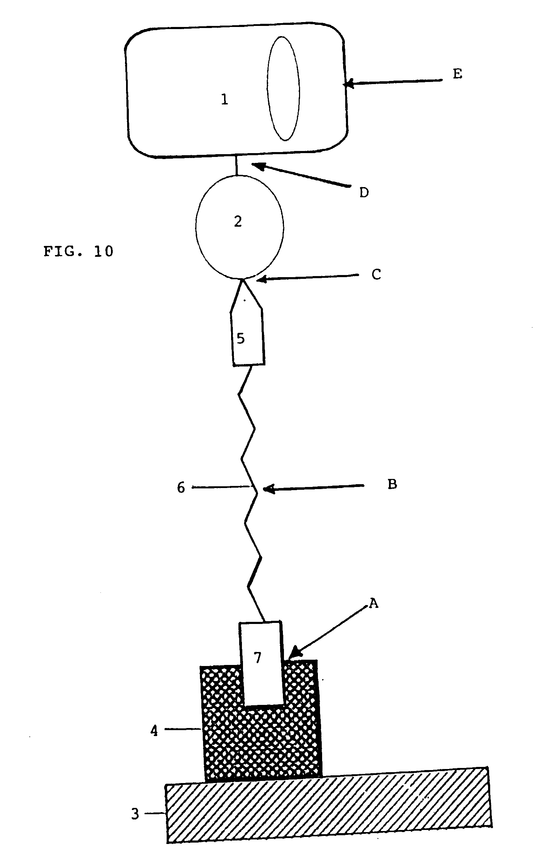

La figure 10 représente de manière schématique un microorganisme recombinant (1) comportant à sa surface une molécule à activité enzymatique (2), immobilisé sur un support solide (3) comportant une protéine complexante (4) telle que l'avidine ou la streptavidine, par un marqueur constitué d'un inhibiteur (5) covalent du site actif de la molécule à activité enzymatique (2), d'un bras (6) et d'un ligand (7) immobilisable par la protéine complexante (4) sur le support solide (3).

Les microorganismes ou tout ou une partie de leur génome peuvent être recueillis:
- en détachant le ligand (7) du support solide avec une solution éluante, telle qu'une solution à un pH de l'ordre de 4 (A);
- en clivant le bras (6) du marqueur (B);
- en clivant la liaison entre l'inhibiteur (5) et la molécule à activité enzymatique (2) (C);
- en clivant le lien peptidique entre le microorganisme (1) et la molécule à activité enzymatique (2) (D);
- en détruisant le microorganisme (1) et en récupérant tout ou une partie de son génome (E).

Le procédé sera décrit plus en détail en référence aux figures annexées, dans les exemples suivants.

### Exemple I: Construction et sélection de phages porteurs de la β-lactamase active (fd-bla)

Les virus présentant à leur surface des molécules à activité enzymatique sont construits à partir de phages "filamenteux".

Les phages filamenteux comme M13 ou fd sont des virus bactériens qui, dans la forme libre, contiennent un ADN circulaire monocaténaire de-6,4 kb encapsulé dans une coque protéique en forme de cylindre très allongé constituée de cinq protéines (Beck & Zink, Gene (1981), 16,-p. 35 à 58; Grant et al., J. Biol. Chem. (1981), 256, p. 539 à 546; Lopez & Webster, Virology (1983), 127, p. 177 à 193).

Parmi celles-ci, le produit du gène III (pIII) est une protéine complexe, présente à raison de 3 à 5 copies, qui 30 ferme une des extrémités du virion et est composée de trois parties: un peptide signal qui sert à exporter la protéine vers le périplasme de la bactérie et est clivé lors de l'excrétion; une partie globulaire nécessaire à l'infection qui reconnaît le pilus F de la cellule cible de E. coli; une 35 partie hydrophobe, transmembranaire pendant la phase de morphogénèse du virus qui assure par la suite l'ancrage de pIII dans la particule (J. Armstrong et al., FEBS Letters (1981), 135, p. 167-172; C.W. Gray et al., J. Mol. Biol. (1981) 146 p. 621 à 627; G. Glasez-Wuttke et al., BBA (1989) 985, p. 239 à 247).

A la suite de leurs travaux sur le rôle et le fonctionnement de la protéine pIII dans la physiologie du phage fd, Smith et Crissman (Virology (1984), 132, p. 445 à 455) ont montré qu'il était possible d'introduire par les méthodes de recombinaison d'ADN, une séquence peptidique étrangère à l'intérieur de la séquence de la protéine pIII (Science (1985) 228, p. 1315 à 1317) ou entre le peptide signal et la séquence de la protéine mature (Gene (1988), 73, p. 305 à 318). Cette insertion n'empêche pas la viabilité du phage et la séquence introduite est accessible.

Le phage-enzyme fd-tet-β-lactamase (fd-bla est construit à partir du phage fdDOG1 (Clackson et al, Nature, 352, p. 624 à 628 (1991) dans lequel on a introduit la séquence codant pour la β-lactamase mutée.

Ce phage est un dérivé du phage fd-tet (Beck et al., Nucl. Acids. Res., 5, p. 4495 à 4503 (1978)), dans lequel un "polylinker" est inséré entre la séquence codant pour le peptide signal de la protéine III (pIII) et la séquence codant pour la pIII mature; de cette manière, la β-lactamase formera avec la pIII une protéine de fusion. Le phage fd-tet est lui-même dérivé du phage fd (Zacher et al., Gene, 9 p. 127 à 140 (1980)) par insertion du gène de résistance à la tétracycline du transposon Tn 10.

Le phage fdDOG1 comprend deux sites de restriction (Not1 et ApaL1) construits par mutations dans la région codant pour la protéine pIII, entre le peptide signal et la partie globulaire.

Le phage fdDOG1 comprend également le gène de la tétracycline inséré dans la zone de l'origine de réplication du phage et permettant une propagation du phage comme plasmide.

### Isolation de la séquence nucléotidique codant pour la β-lactamase

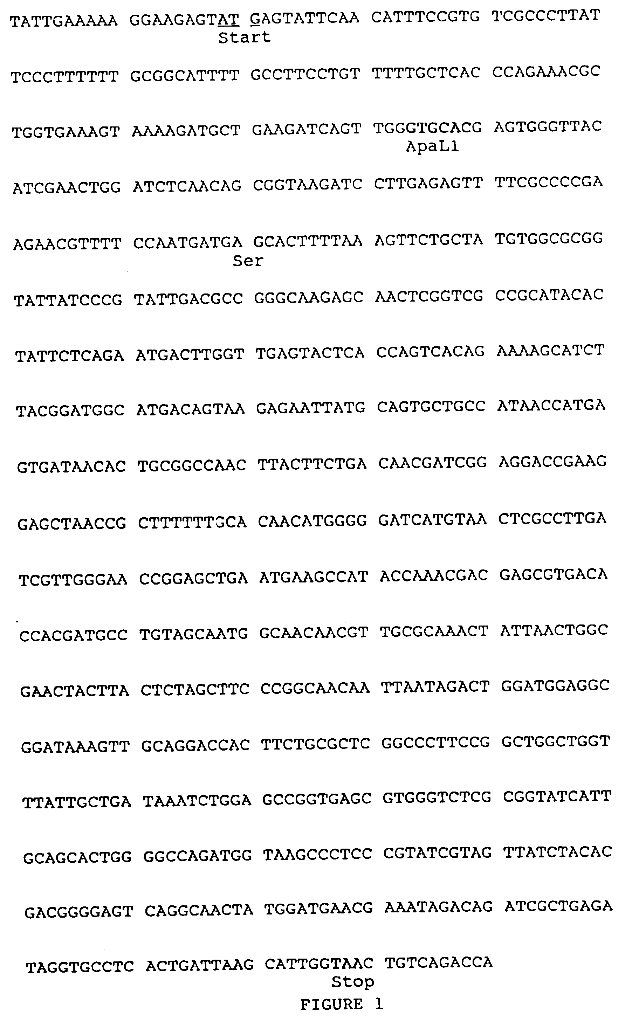

Le gène de la β-lactamase du plasmide pBR322 de E. coli (dont la séquence est donnée par Sutcliffe, PNAS USA, 75, p. 3737 à 3741 (1987) et à la figure 1) a été isolé à partir du phagemide pBluescript S/K+®.

Etant donné que le clonage dans fdDOG1 utilisera le site ApaLl et que la séquence de la β-lactamase contient un tel site, il faut d'abord enlever celui-ci par mutagénèse dirigée. Cette mutation conserve la séquence protéique.

Le phagemide pBluescript S/K+® (de la firme Stratagène®, vendu par Ozyme®) est muté selon la méthode de Eckstein (Nucl. Acids Res., 14, p. 9679 à 9698 (1986) à l'aide d'un kit commercialisé par la firme Amersham®.

Après la mutagénèse in vitro, le double brin du phagemide pBluescript S/K+® muté (pBluescript-M) est utilisé pour transformer la souche TG1 de E. coli (Gibson, Studies on the Epstein-Barr virus genome, PhD Thesis, Cambridge University England (1984).

Les cellules transformées sont étalées sur milieu LB-Amp (Sambrook et al., Molecular Cloning, A laboratory manual, second edition, Cold Spring Harbor Laboratory Press (1989) Appendice A).

Des colonies sont récupérées et cultivées en milieu LB-Amp. La forme simple brin de pBluescript-M est produite par addition au milieu du "phage-helper" R408 (Russel et al., 45, p. 333 à 338 (1986)).

Le brin pBluescript-M est ensuite séquencé selon la méthode de Sanger (PNAS USA, 74, p. 5463 à 5467 (1977)) à l'aide de l'amorce 5'-TTTAAAAGTGGCCATCATTGGA centrée à la position codant pour Ser 68 afin de vérifier que, dans les clones choisis, la mutation est effectivement introduite.

### Clonage de la β-lactamase dans le phage fdDOG1

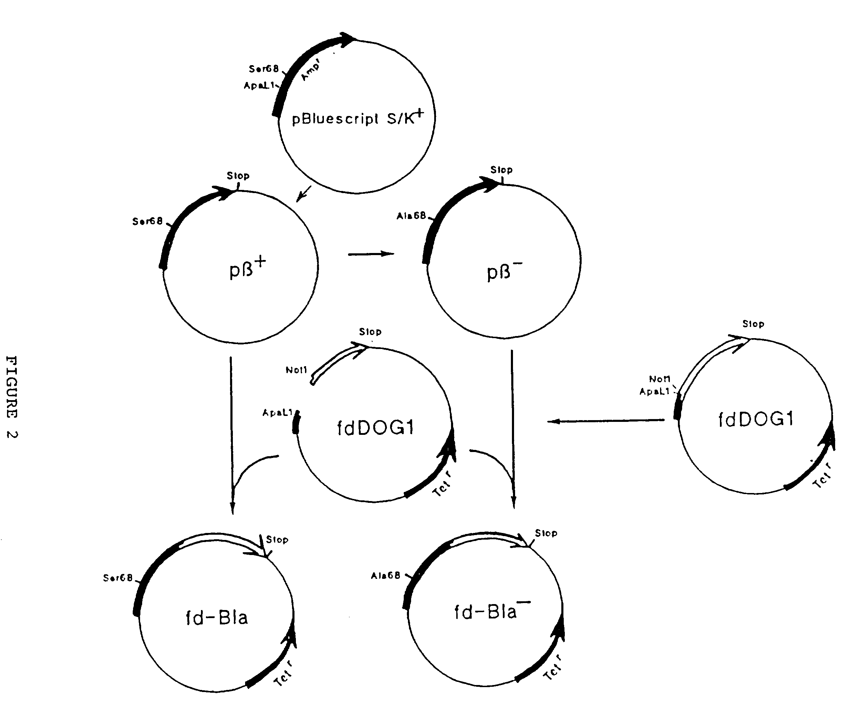

La séquence du gène codant pour la β-lactamase mature est insérée dans le phage fdDOG1 entre la séquence codant pour le peptide signal de la pIII et celle codant pour la pIII mature. On utilise les sites de clonage ApaLl et Notl selon le schéma donné à la figure 2. A cet effet, le gène de la β-lactamase est récupéré du phagemide pBluescript-M avec introduction des sites de restriction adéquats par PCR.

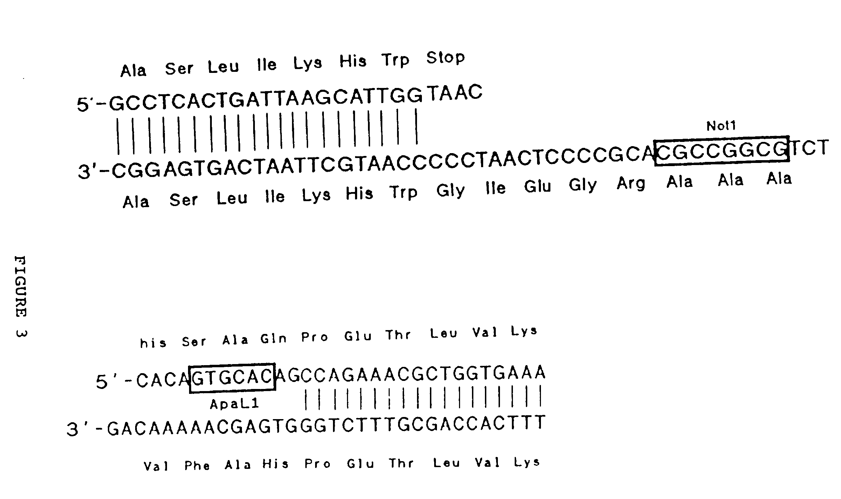

Les amorces utilisées montrées à la figure 3 contiennent les caractéristiques suivantes:

L'amorce en 5' est un 30mère complémentaire sur 18 bases du gène de la β-lactamase mature à partir du deuxième codon (codant pour Pro 2). L'extension 5' (12 bases) introduit le site ApaL1 et provoque une mutation qui a pour conséquence le remplacement de His 1 en Gln 1. Cette mutation est introduite pour éviter que les phages ne portent une β-lactamase ayant la séquence amino terminale His-Pro-Gln susceptible de présenter une affinité pour l'avidine ou la streptavidine (Devlin et al. Science, 249, p. 404 à 406 (1990)) utilisée ultérieurement pour leur sélection.

L'amorce en 3' est un 47mère complémentaire sur 21 bases de la fin du gène de la β-lactamase. L'extension remplace le codon stop par un codon codant pour une glycine, introduit quatre codons entre le gène codant pour la β-lactamase et le gène codant pour la protéine pIII, ainsi que le site de la restriction Notl pour le clonage. Ces quatre acides aminés supplémentaires constituent un site de clivage potentiel entre les deux protéines pour le facteur Xa. La protéine pIII assure, en effet, la capacité d'infection des phages (Goldsmith et al., Biochemistry, 16, p. 2686-2694 (1977). Si la présence d'une protéine aussi volumineuse que la β-lactamase affecte la capacité d'infection, une incubation avec le facteur Xa permet de restaurer celle-ci.

En outre, ce site constitue un point de clivage supplémentaire pour récupérer les microorganismes.

Le phage fdDOG1 est digéré pendant 18 heures avec ApaL1 et Not1 et purifié sur gel d'agarose. Par ailleurs, la séquence du phagémide pBluescript-M est amplifié avec les amorces décrites ci-dessus par 34 cycles de PCR (1 min. à 95°C, 1 min. à 60°C et 2,5 min. à 72°C) en présence de Taq-polymérase® (Saiki et al., Science, 239, p. 487 (1988). Après purification sur gel, le produit est digéré par ApaL1 et Not1 pendant 18h et repurifié; ensuite 1,5 µq de fdDOG1 digéré et 250 ng du gène de la β-lactamase sont mélangés dans 60 µl avec 4 unités de T4 DNA ligase (Gibco-BAL®) et maintenus une nuit à 16°C. Le mélange de ligation purifié est utilisé pour transformer la souche TG1 de E.Coli.

Les cellules sont étalées sur milieu LB-tet et LB-Amp (Sambrook et al.) où l'on observe que la présence du phage assure la résistance à l'antibiotique. Des colonies sont alors cultivées en milieu 2xYT-Tet ou en milieu 2xYT-Amp pour préparer le phage fd-bla.

Au lieu de construire des phages-enzymes par manipulation de la protéine pIII, il est également possible de construire une protéine de fusion avec la protéine pVIII (1) qui est une petite protéine de 5,2 kd, constituant le corps du cylindre du phage fd et qui est normalement présente en 2700 exemplaires (Newman et al., J. Mol. Biol (1977), 116, p. 593 à 606). Si la taille de la molécule d'ADN est modifiée, le nombre de copies de la protéine pVIII peut etre ajustée pour inclure celle-ci.

Cette variante est réalisée avec un phagemide de manière à pouvoir contrôler le nombre de copies de la protéine de fusion Kang et al., (1991) Proc. Natl. Acad. Sci. USA, vol. 88, p. 4363-4366.

### Caractérisation du phagre porteur de la β-lactamase active (fd-bla)

Le phage ainsi construit est caractérisé de la manière suivante. On met d'abord en évidence le fait que l'infection par le phage confère aux cellules une résistance à l'ampicilline. Ensuite on montre directement que le phage lui-même présente une activité d'hydrolyse d'un β-lactame, la nitrocéfine. Le fait que l'activité enzymatique ne se retrouve pas dans l'ultrafiltrat après filtration sur une membrane qui laisse passer la β-lactamase, indique déjà que l'enzyme est liée au phage. Enfin, on analyse par électrophorèse sur gel et "western blot" les protéines du phage et on montre que la protéine pIII se retrouve sous forme d'une protéine de fusion avec la β-lactamase. Ceci confirme que la construction est bien correcte et que, pendant la morphogénèse du virus, il n'y a pas de clivage protéolytique qui aboutirait au détachement de la β-lactamase du phage.

Par infection de cellules d'E. Coli TG1 avec ce phage et étalement sur milieu LB-Amp, on obtient des colonies résistantes à l'ampicilline. L'activité β-lactamase est directement détectée sur boîte de Pétri par un test à la nitrocéfine (Plückthun et al., J. Biol. Chem., 262, p. 3951 à 3957 (1987)). De même le phage isolé du milieu de culture après centrifugation des cellules, précipité au PEG à 4%, 0,5M NaCl (final) et resuspendu en tampon tris 50mM, NaCl 150 mM à pH 7,5, à une concentration de l'ordre de 10¹² unités transductrices (tu) par ml, hydrolyse la nitrocéfine.

L'activité spécifique de l'enzyme portée par les phages était identique à celle de la β-lactamase RTEM en solution (Boehringer). La mesure de l'activité a aussi été réalisée après l'ultrafiltration des phages sur membrane Centri/por présentant une barrière d'exclusion à un PM de 100.000 qui laisse passer la β-lactamase RTEM commerciale. Moins de 5% de l'activité β-lactamase a été détectée dans l'ultrafiltrat ce qui indique que l'enzyme reste attachée au phage.

Des virus fd-tet et fd-bla sont préparés et concentrés (10¹² tu/ml). 20 µl de chaque solution sont chargés sur un gel de polyacrylamide 12,5%-SDS et analysés (miniprotean® II de Biorad®). Par "Western blot", les protéines sont transférées sur nitrocellulose (minitransblot® de Biorad®) et la révélation est effectuée avec un anticorps de lapin anti-protéine III, suivi d'un anticorps de chèvre contre un anticorps de lapin couplé à la HRP (horse radish peroxidase). Le substrat chromophore de la HRP révèle des bandes de PM apparent 70 kd et 105 kd pour fd-tet et fd-bla respectivement. On a aussi effectué la révélation avec un anticorps anti-β-lactamase, on trouve une bande uniquement pour fd-bla à l'emplacement prévu pour la protéine de fusion.

### Exemple II: Construction et caractérisation de phages porteurs d'une β-lactamase inactive (fd-bla⁻)

Afin de tester l'efficacité du procédé de sélection selon l'invention, on procède à la construction et à la caractérisation de phages porteurs d'une β-lactamase inactive (fd-bla⁻).

Le phagemide pBluescript-M est soumis à une mutagénèse dans laquelle le codon codant pour la sérine 68 essentielle est muté en alanine en vue d'inactiver l'enzyme.

Le gène muté est récupéré selon la même technique que celle employée pour la β-lactamase active (amplification par PCR avec introduction de sites de restriction (cfr. figure 2)). Le phage porteur de β-lactamase inactive (fd-bla-) est caractérisé par analyse de fragments de restriction, séquençage complet du gène Bla, recherche d'activité enzymatique et analyse des protéines.

La mutation S68A est introduite selon le même protocole que la mutation du site ApaLl. Les cellules transformées sont étalées sur milieu X-Gal (Horwitz et al., J. Med. Chem., 7, p. 574 (1964)) sans ampicilline. Les colonies bleues sont cultivées en milieu 2xYT et la forme simple brin est produite par addition du "phage-helper" R408. La mutation est séquencée par la méthode de Sanger à l'aide de l'amorce 5'-TAGTGTATGCGGCGACC complémentaire de la région codant pour G90-Y95.

Après amplification du gène par PCR, clonage, transformation et étalement sur milieu tet comme cela a été réalisé pour la β-lactamase active, des colonies sont récupérées et cultivées sur milieu 2xYT-tet. L'ADN double brin du phage est isolé et digéré par les nucléases de restriction BamB1 et EcoR1. Les produits de digestion sont séparés sur agarose; les transformants montrent 2 fragments de 4,7 kpb et de 5,5 kpb, en accord avec ce qui est attendu. Le séquençage complet du gène Bla muté est réalisé avec des amorces 5'-TAGTGTATGCGGCGACC, 5'-AAGGCGAGTTACATG, 5'-CCAGCCAGCCGGAAG et 5'-TGCTAAACAACTTTC localisées respectivement dans les régions codant pour G90-Y65, H156-L160, L223-W227 de la β-lactamase et E5-A9 de la protéine pIII mature). On vérifie ainsi que l'amplification par PCR n'a pas introduit d'autres mutations que celle visant à l'inactivation du site actif.

On cherche ensuite la présence d'une activité β-lactamase en réalisant, sur le surnageant d'une culture de phage fd-bla-, un test à la nitrocéfine comme décrit plus haut. En accord avec ce qui est attendu, aucune activité n'est observée.

Enfin l'analyse des protéines de manteau du phage par électrophorèse sur gel dénaturant, "western blot" et détection immunologique est réalisée comme pour le phage fd-bla. La protéine de fusion PIII-β-LACTAMASe est correctement mise en évidence.

### Exemple III: Synthèse du marqueur de-la β-lactamase active pourvu d'un bras de faible taille entre inhibiteur suicide et ligand

Les phages dont l'ADN comporte le gène de la β-lactamase active et qui sont porteurs de l'enzyme correspondante attachée à la protéine de manteau pIII seront sélectionnés par une réaction avec le marqueur selon l'invention. Ce marqueur consiste en une molécule bifonctionnelle qui comporte à une extrémité un inhibiteur covalent de la β-lactamase et à l'autre un ligand en l'occurrence la biotine, facilement immobilisable sur un support solide.

Le module inhibiteur suicide est constitué d'un sulfone de péname (voir Fisher et al., Biochemistry (1981), 20, 2726-2731, Mezes et al., FEBS Let. (1982), 143, 265-267, Clarke et al., (1983), 748, 389-397, Dmitrienko et al., Bioorg. Chem. (1985), 13, 34-46) fonctionnalisé de manière à permettre son attachement par un bras à un ligand ou un support.

L'imino-biotine peut être également avantageusement utilisée car son affinité pour la streptavidine peut être considérablement réduite en diminuant le pH de la solution. Ceci nous donnera une possibilité supplémentaire pour isoler les phages du support après immobilisation.

Ces deux entités sont reliées par un bras de longueur adéquate pour permettre l'interaction du ligand porté par le marqueur avec la protéine immobilisée.

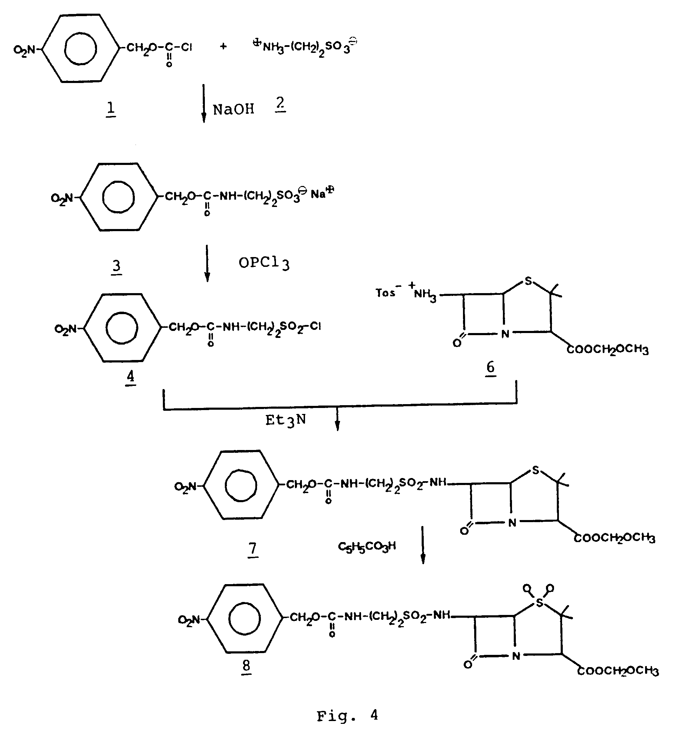

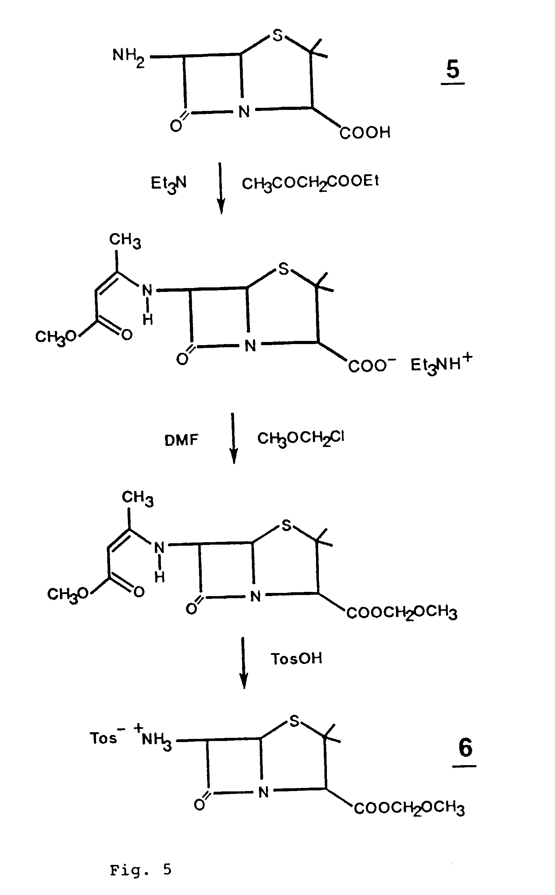

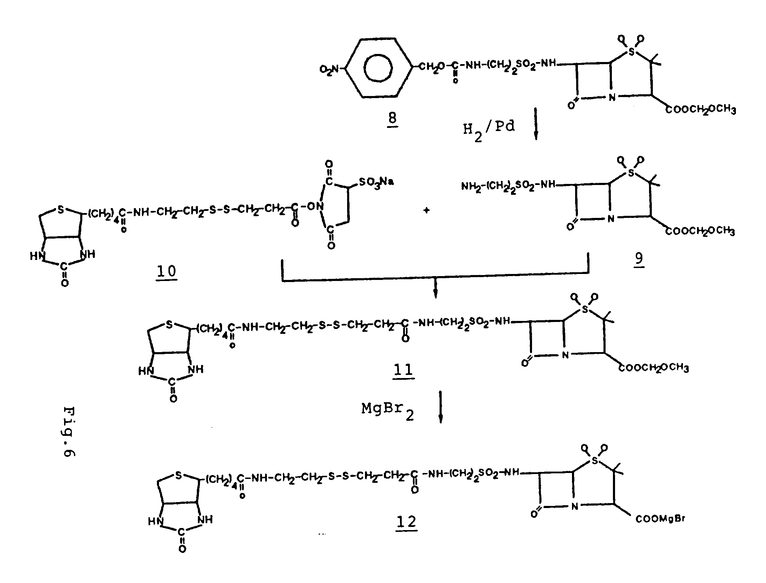

Le schéma de synthèse de la molécule bifonctionnelle est donné aux figures 4 à 6. La plupart des étapes de cette synthèse sont décrites dans la littérature chimique ou font intervenir des modes opératoires classiques. Les molécules sont caractérisées par spectroscopie IR et 1H-RMN.

Le p-nitrobenzyloxycarbonyle-2-aminoéthane-sulfonate de sodium (3) est préparé par addition de deux équivalents de chloroformiate de p-nitro benzyle à la taurine (2) dissoute un équivalent de NaOH 1N à 0°C en neutralisant continuellement le milieu par addition de NaOH, dans les conditions de Schotten-Baumann. Après extraction de l'alcool p-nitrobenzylique à l'acétate d'éthyle, on a lyophilisé le produit. Le rendement était de 66%.

Le sulfonate (3) a été transformé en chlorure de sulfonyle (4) avec l'oxychlorure de phosphore selon la méthode de Fujita (Synthesis, 423-424 (1982)). Par lavage de l'huile obtenue avec de l'éther sec, on a obtenu le produit sous forme d'une poudre blanche avec un rendement de 97%.

Parallèlement l'acide 6-aminopenicillanique (5) a été transformé en son ester de méthoxyméthyle (6) selon la méthode de Manhas et al. (Synthesis, 549-552 (1983)) à l'aide de chlorure de méthoxyméthyle après protection transitoire à l'acétoacétate d'éthyle.

Le chlorure d'acide (4) est couplé avec l'ester de l'acide 6-aminopénicillanique (5) dans le dichlorométhane en présence de triéthylamine. Le produit (7) purifié sur colonne de silice (éluant: acétate d'éthyle-dichlorométhane 80:20) a été obtenu avec un rendement de 50%.

Ce produit a été oxydé en sulfone (8) selon la méthode de Johnson et al. (J. Org. Chem., 28, 1927-28 (1963)); le produit obtenu a été lavé à l'éther pour donner un blanc solide qui a été purifié sur silice (éluant: acétate d'éthyle-dichlorométhane 50:50). Le rendement était de 75%.

Par déprotection de la fonction amine par hydrogénation sur palladium/carbone selon un procédé classique, on a obtenu le produit (9) que l'on a couplé directement avec le sulfosuccinimidyl 2-(biotinamido) éthyl-1,3-dithiopropionate (10) (commercialisé par PIERCE®), pour obtenir le précurseur du marqueur (11) qui a été purifié par chromatographie sur silice (éluant: acétate d'éthyle-dichlorométhane 0 50:50). Le rendement était de 25%.

La protection de la fonction carboxylique en position 3 du cycle péname dans (11) a été enlevée à l'aide de bromure de magnésium dans le dichlorométhane selon la méthode de Kim et al. (Tetrahedron Letters, 32, 3099-3100(1991)). Le 15 produit brut récupéré (12) après évaporation du solvant a été utilisé directement pour inhiber l'enzyme et marquer les phages porteurs d'enzyme active.

### Exemple IV: Immobilisation des phages porteurs de la β-lactamase active (fd-bla)

L'utilisation d'un marqueur covalent fixant de manière irréversible le site actif de l'enzyme et rendant toute réutilisation de l'enzyme impossible, ne constitue pas un inconvénient dans le procédé de sélection de l'enzyme. En effet, dans ce procédé on désire seulement sélectionner et recueillir la séquence génétique codant pour la molécule active.

On détermine d'abord les conditions optimales d'inhibition par le marqueur bifonctionnel des phages porteurs de la β-lactamase active. A cet effet, on mesure, par le test à la nitrocéfine, la vitesse de disparition de l'activité β-lactamase des phages en fonction du temps après addition de l'inhibiteur. On incube alors des phages en présence du marqueur.

Ensuite, le produit de réaction est passé sur une colonne de streptavidine couplée à de l'agarose. Les phages sont alors élués par clivage de la fonction disulfure du marqueur. Ils servent à réinfecter une culture de E. coli. A partir de cette culture, le gène codant pour la β-lactamase active est aisément récupérable.

Une culture de bactéries infectées par les phages fd-bla est préparée dans un milieu 2xYT contenant 15 µg par ml de tétracycline et incubée sous agitation à 37°C pendant 16 heures. Après centrifugation de la culture pendant 10 minutes à 10000 tpm (rotation par minute), on récupère le surnageant. Les phages sont précipités par addition de 1/5 volume de solution de polyéthylène glycol à 20%, 2,5M en NaCl. Après incubation pendant 1 heure à 4°C et centrifugation, les phages sont resuspendus dans du tampon acétate 50 mM à pH 5,0.

Le marqueur (12) en solution dans le DMSO, à une concentration de 1 mg/25µl a été ajoutée à une concentration finale de 0,25 mM à la solution contenant les phages (approximativement 10¹¹ tu/ml) et 1% de BSA. L'inhibition est poursuivie pendant 30 minutes.

Par ailleurs, une suspension de 250 µl d'agarose-streptavidine (PIERCE®) est lavée avec 20 ml de tampon PBS contenant 1% de BSA (Bovine Serum Albumine, Sigma). 0,3 ml de la solution contenant 1,25 x 10⁹ phages, a été chargée sur la colonne, lavée avec 6 ml de PBS/1% BSA et 6 ml de PBS. Les phages sont ensuite élués avec 1 ml d'une solution de dithiothréitol 500 mM dans un tampon PBS. Un aliquot de phages récupérés a été incubé après changement de tampon avec 10 ml de cellules de E. coli TG1 en croissance exponentielle à 37°C. La récupération des phages a été mise en évidence par étalement de diverses dilutions sur milieu LB-tet et comptage des colonies. On a récupéré de cette manière 1,8 x 10⁴ phages porteurs de β-lactamase active, soit 14,4 ppm.

### Exemple V: Immobilisation de phages porteurs de β-lactamase inactive (fd-bla⁻)

1,05 x 10⁹ phages (fd-bla⁻) contenus dans 0,3 ml de tampon acétate à pH 5, ont été mis en présence du marqueur (12) selon l'invention et le produit de réaction a été passé sur une colonne de streptavidine couplée à de l'agarose et élué selon un protocole identique à celui décrit dans l'exemple 4. On a récupéré dans ce cas 2,0 x 10³ phages porteurs de β-lactamase inactive soit 1,9 ppm.

La comparaison des résultats des exemples IV et V montre que les phages actifs sont mieux retenus que les phages inactifs par un facteur 8.

### Exemple VI: Enrichissement en phages actifs d'un mélange contenant des phages actifs (fd-bla) et inactifs (fd-bla⁻): élution par le dithiothréitol

Une expérience du même type a été réalisée avec un mélange de phages actifs et de phages inactifs pour déterminer directement le facteur d'enrichissement en phages actifs.

Un mélange contenant 6,4 x 10⁹ phages actifs et 1,4 x 10¹⁰ phages inactifs dans 0,3 ml de tampon acétique à pH 5 en présence de 1% BSA a été mis en présence du marqueur (12) à une concentration finale de 0,25mM. L'excès de marqueur a été éliminé par ultrafiltration sur une unité Ultrafree 30,000 de Millipore®. La solution a été chargée sur streptavidine-agarose. Après lavage de la colonne par 5 ml de PBS/1% BSA, les phages ont été élués par du dithiothréitol 500 mM. Un aliquot des phages élués a été étalé à diverses dilutions sur boite LB-tet; les colonies ont été ainsi été comptées. On a récupéré de cette manière un total de 1,3 x 10⁶ phages. Le nombre de phages porteurs de β-lactamase active a été déterminé en mesurant directement l'activité β-lactamase des colonies par le test à la nitrocéfine sur boite comme décrit plus haut. Ce nombre a aussi été vérifié par étalement d'un aliquot de l'éluat sur boite LB-Amp. Le nombre de phages actifs élués était de 1,1 x 10⁶. Le facteur d'enrichissement obtenu était donc de 5,5.

### Exemple VII: Enrichissement en phages actifs d'un mélange contenant des phages actifs (fd-bla) et inactifs (fd-bla⁻): élution par le facteur Xa

Une expérience du même type a été réalisée avec un mélange de phages actifs et de phages inactifs pour déterminer directement le facteur d'enrichissement. L'élution a cette fois été réalisée avec le facteur Xa clivant un lien peptidique entre la β-lactamase et la protéine III.

Un mélange contenant 2,8 x 10¹⁰ phages actifs et 2,2 x 10¹⁰ phages inactifs dans 1,5 ml de tampon acétique 1% BSA a été mis en présence du marqueur (12) à une concentration finale de 0,75 mM, passé sur streptavidine-agarose. Après lavage de la colonne avec 80 ml de PBS, 2 ml de tampon TRIS 0,1 M à pH 7 contenant 0,02% de NaN₃ et 2 ml de tampon Tris à pH 7, on a additionné à la suspension 1 ml d'une solution contenant 10 µg de facteur Xa (Boeringher) et laissé une nuit à température de chambre sous faible agitation. Après centrifugation, le surnageant a été analysé par étalement à diverses dilutions sur boîte LB-tet; les colonies ont ainsi été comptées. On a récupéré de cette manière un total de 6 x 10⁵ phages. Le nombre de phages actifs élués était de 5,7 x 10⁵. Le facteur d'enrichissement obtenu était donc de 15.

### Exemple VIII: Enrichissement en phages actifs d'un mélange contenant des phages actifs (fd-bla) et inactifs (fd-bla⁻): préimmobilisation du marqueur

Une autre expérience a été réalisée avec un mélange de phages actifs et de phages inactifs, mais avec immobilisation préalable du marqueur sur la colonne de streptavidine-agarose.

250 µl de suspension de streptavidine-agarose sont incubés pendant une heure dans un tampon acétique à pH 5 en présence de 1%: de BSA; après lavage avec 2,5 ml de tampon acétique sans BSA, on ajoute 2 µl d'une solution 50 mM de marqueur (12) dans le DMSO. Après 1 minute et 5 cycles de lavage et centrifugation avec chaque fois 1 ml de tampon acétique, 0,8 ml de phages dans un tampon acétique 1% BSA contenant 9,6 x 10¹⁰ phages actifs et 5 x 10¹⁰ phages inactifs ont été ajoutés à la suspension sous faible agitation pendant une nuit à 4°C. Après lavage par 20 ml de PBS/1% BSA et 50 ml de solution 0,1% de Tween 20 dans PBS, les phages sont élués avec le facteur Xa et comptés comme dans l'exemple VI. On a récupéré de cette manière 1,15 x 10⁵ phages actifs et 1,85 x 10⁵ phages inactifs. Le facteur d'enrichissement obtenu tait donc de 3,2.

### Exemple IX: Enrichissement en phages actifs d'un mélange contenant des phages actifs (fd-bla) et inactifs (fd-bla⁻): élution par hydrolyse du lien acyl-enzyme

Une expérience analogue à celle de l'exemple VIII a été réalisée avec un mélange de phages actifs et de phages inactifs pour vérifier la possibilité de récupération des phages par clivage du lien entre la β-lactamase et l'inhibiteur suicide, à savoir le lien ester de l'acyl enzyme. Ce clivage a été réalisé par hydrolyse.

Un mélange contenant 2,8 x 10¹⁰ phages actifs et 1,05 x 10¹⁰ phages inactifs a été chargé sur une colonne où le marqueur avait été immobilisé. Après lavage extensif comme dans l'exemple VIII, on a laissé la suspension une nuit à température de chambre et analysé le surnageant d'une centrifugation. On a récupéré de cette manière 1,4 x 10⁵ phages actifs et 2,5 x 10⁵ phages inactifs. Le facteur d'enrichissement obtenu était donc de 2,1.

Dans les exemples mentionnés, le pourcentage de phages immobilisés est faible parce que la taille du bras entre le ligand immobilisable et l'inhibiteur est assez faible. Pour éviter cet inconvénient, un autre marqueur possédant un bras de plus grande taille a été préparé.

### Exemple X: Synthèse d'un marqueur de β-lactamase active pourvu d'un bras de grande taille entre inhibiteur suicide et ligand

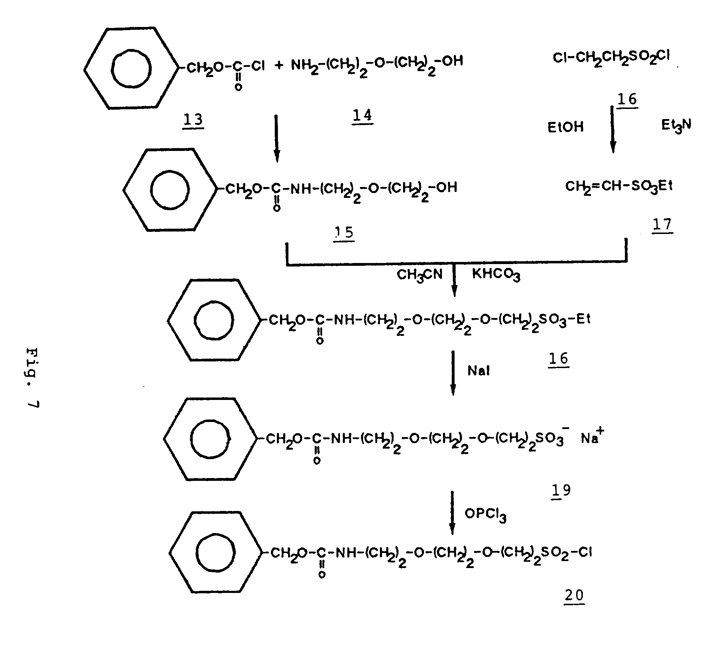

Le carbobenzyloxy-2-(2-aminoéthoxy)-éthanol (15) (figure 7) a été préparé par addition de 1 équivalent de chloroformiate de benzyle (13) à du 2-(2-aminoéthoxy)-éthanol (14) dans l'eau saturée en bicarbonate. Le produit a été extrait en phase organique et purifié sur colonne de silice (éluant: acétate d'éthyle-dichlorométhane 50:50). Le rendement était de 58%.

Le vinyl-sulfonate d'éthyle (17) a été préparé à partir de chlorure de 2-chloroéthane-sulfonyle (16) selon la méthode de Whitmore et Landau (J. Am. Chem. Soc., 68, p. 1797-1798 (1946)). Il a été purifié par distillation horizontale à 88°C sous 0,6 mm de mercure pour donner une huile incolore (rendement 45%). Un équivalent de l'aminoalcool protégé (15) a été additionné sur (17) dans l'acétonitrile sec auquel a été ajouté du bicarbonate de potassium finement broyé. Après chauffage, pendant 3 jours à 80°C, filtration et évaporation du solvant, le produit (18) a été purifié sur silice (éluant: acétate d'éthyle-dichlorométhane 50:50). Le rendement était de 60%. La fonction sulfonique a été déprotégée selon la méthode de Tipson et al. (J. Org. Chem., 12, 133-137 (1947)) pour donner après 24 heures de réaction (19) sous forme d'une poudre blanche après lavage à l'hexane (rendement 86%). Ce produit a été transformé en chlorure d'acide (20) selon la méthode de Fujita (Synthesis, 423-424 (1982)).

Le chlorure d'acide (20) (figure 7) a été couplé directement avec l'ester de d'acide 6-aminopénicillanique (6) dans le dichlorométhane en présence de triéthylamine pendant une heure. Le produit obtenu (21), purifié sur silice (éluant: acétate d'éthyle-dichlorométhane 50:50) a été obtenu avec un rendement de 34%. Il a été oxydé en sulfone (22) selon la méthode de Johnson et al. (J. Org. Chem., 28, 1927-28 (1963)), le produit obtenu a été lavé à l'éther pour donner un solide blanc qui a été purifié sur silice (éluant: acétate d'éthyle-dichlorométhane 50:50). Le rendement était de 72%.

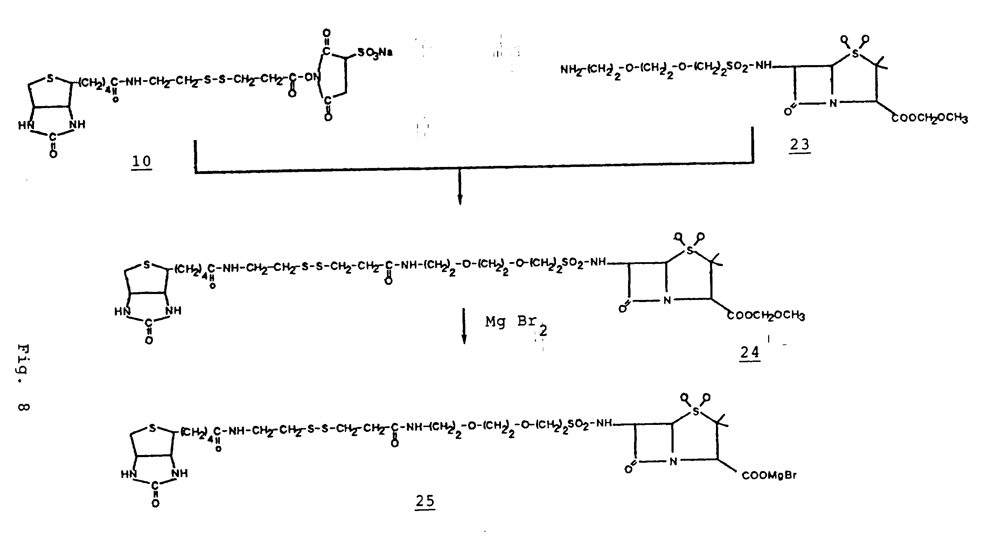

Par déprotection de la fonction amine par hydrogénation sur palladium/carbone dans l'éthanol selon le procédé classique, on a obtenu le produit (23) (figure 8) que l'on a couplé directement avec le sulfosuccinimidyl 2-(biotinamido) éthyl-1,3-dithiopropionate (10) (commercialisé par PIERCE®) pour obtenir le précurseur du marqueur (24) qui a été purifié par chromatographie sur silice (éluant: acétate d'éthyle-dichlorométhane 50:50). Le rendement était de 13%

La protection de la fonction carboxylique en position 3 du cycle péname dans (24) a été enlevée à l'aide de bromure de magnésium dans le dichlorométhane selon la méthode de Kim et al. (Tetrahedron Letters, 32, 3099-3100 (1991)).

### Exemple XI: Enrichissement en phages actifs d'un mélange contenant des phages actifs (fd-bla) et inactifs (fd-bla⁻): marquage par l'inhibiteur (24) élution par le facteur Xa

Une expérience d'enrichissement a été réalisée avec marquage par l'inhibiteur (24) d'un mélange de phages actifs et de phages inactifs pour déterminer l'effet de la taille du bras reliant le ligand biotine avec le module inhibiteur suicide.

Un mélange contenant 1,2 x 10¹⁰ phages actifs et 3,3 x 10¹⁰ phages inactifs dans 1,5 ml de tampon acétique 1%BSA a été mis en présence du marqueur à une concentration finale de 0,75 mM, passé sur streptavidine-agarose. Après lavage de la colonne, élution comme décrit dans l'exemple VII et analyse, on a trouvé 6,8 x 10⁶ phages actifs et 1,9 x 10⁵ phages inactifs. Le facteur d'enrichissement obtenu était donc de 98.

### EXEMPLE XII: Synthèse d'un deuxième marqueur de β-lactamase active pourvu d'un bras de grande taille entre inhibiteur suicide et ligand

En vue de disposer d'une méthode générale de synthèse de marqueur pourvu d'un bras de grande taille entre le ligand et la tête inhibitrice, un précurseur porteur d'une fonction ester activé et donc couplable à de nombreux inhibiteurs et comportant lui-même un bras de grande taille a été préparé. Il a été couplé à l'inhibiteur suicide de la β-lactamase.

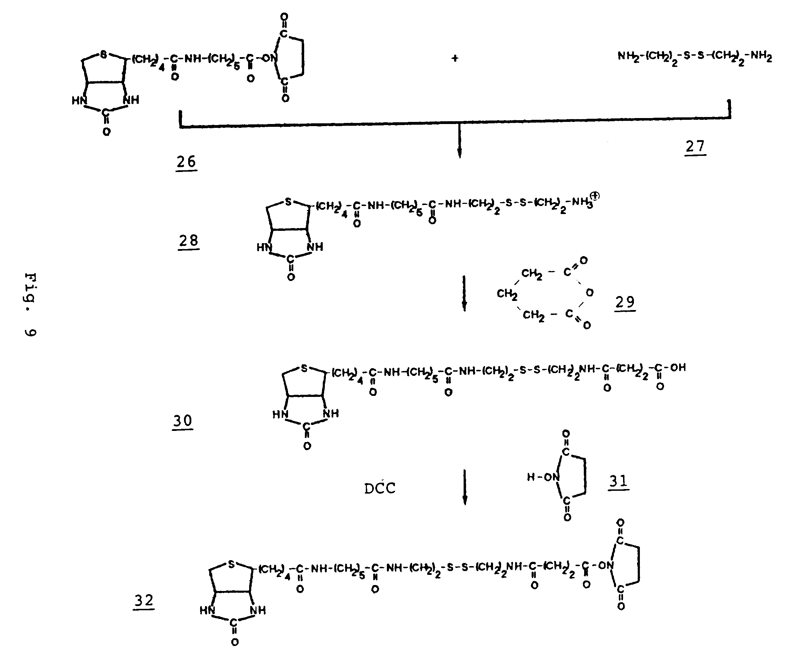

L'ester activé de la N-hydroxysuccinimide avec la biotine-N-ε-aminocaproique (26) a été préparé comme décrit (Wilchek et Bayer, Methods Enz. (1990), 184, 123-160). Il a été dissout dans la DMF et ajouté à une solution de 3 équivalents de cystamine (26) dans la DMF (figure 9).

Le produit brut, isolé par précipitation à l'éther (10 fois le volume de la solution) a été purifié sur colonne S Sépharose (Pharmacia®) équilibrée au chlorure de lithium avec comme éluant de l'éthanol à 25%. Le produit d'addition (28) a été élué par du LiCl 50 mM. La solution a été lyophilisée. Le rendement était de 79%.

Trois équivalents d'anhydride glutarique (29) dissous dans l'acétonitrile, ont été ajoutés par portion au produit purifié (contenant du LiCl) dissout à une concentration de l'ordre de 0,01 M en milieu aqueux tamponné (KHCO₃-K₂CO₃, 50/50, 10 mM à pH 9,6) contenant 10% d'éthanol. L'acide obtenu (30) a été précipité par acidification avec HCl 1N jusqu'à pH 2. Le produit a été filtré et séché, le rendement était de 43%.

L'acide (30) a été transformé en ester activé dans les conditions standards par couplage avec la N-hydroxysuccinimide (31) à l'aide de la dicyclohexylcarbodiimide (DCC) dans la DMF. Après 72 heures, le milieu réactionnel a été précipité à l'éther sec. Le précipité a été séché au dessiccateur (rendement: 87%), ce produit (32) a été utilisé tel quel pour la réaction de couplage avec l'ester de l'acide 6-aminopénicillanique déprotégé (6) dans l'éthanol sec.

### Exemple XIII: Enrichissement en phages actifs d'un mélange contenant des phages actifs (fd-bla) et inactifs (fd-bla⁻): immobilisation sur des particules paramagnétiques

On réalise une expérience analogue à l'expérience VI à la différence près que la streptavidine est portée par des particules paramagnétiques. Les lavages sont réalisés par resuspension dans du tampon frais. L'élution des phages immobilisés est réalisée avec du dithiothréitol 50 mM. Le facteur d'enrichissement est déterminé comme dans l'exemple VI.

### Exemple XIV: Enrichissement en phages actifs d'un mélange contenant des phages actifs (fd-bla) et inactifs (fd-bla⁻): immobilisation par "panning" dans un tube en polystyrène

On réalise une expérience analogue à l'expérience VII à la différence près que la streptavidine est immobilisée par "panning" à la surface d'un tube de polystyrène suivant une technique classiquement utilisée pour les tests ELISA. Les lavages sont réalisés par resuspension dans du tampon frais. L'élution des phages immobilisés est réalisée avec du facteur Xa. Le facteur d'enrichissement est déterminé comme dans l'exemple VII.

Etant donné que dans les exemples XIII et XIV, les lavages peuvent être réalisés plus rapidement que dans les exemples utilisant la technique chromatographique et que les phages moins aisément entrappés ici que dans un gel, ces variantes des expériences d'enrichissement sont plus favorables à l'observation de facteurs d'enrichissement élevés.

### Exemple XV: Sélection des phages porteurs d'une abzyme

Dans ce cas, l'inhibiteur covalent sera constitué par un inhibiteur suicide caractéristique de la réaction à catalyser par l'abzyme ou par un analogue de l'état de transition spécifique.

La détection et la sélection de ces phages s'effectue ensuite de manière identique à la détection et la sélection des phages exposant à leur surface des enzymes actives.

## Revendications

1. Procédé de sélection de microorganismes recombinants comportant à leur surface au moins une molécule à activité enzymatique caractérisé en ce que les microorganismes recombinants sont immobilisés sur un support solide, par un inhibiteur covalent du site actif de la molécule à activité enzymatique, relié par un bras au support solide; en ce que les microorganismes recombinants ou tout ou une partie de leur génome sont recueillis et en ce que tout ou une partie de leur génome est isolé.

2. Procédé de sélection selon la revendication 1 caractérisé en ce que préalablement à leur immobilisation sur le support solide, les microorganismes recombinants sont marqués par l'inhibiteur covalent du site actif de la molécule à activité enzymatique, relié par un bras à un ligand immobilisable sur le support solide.

3. Procédé de sélection selon l'une quelconque des revendications 1 ou 2 caractérisé en ce que les microorganismes sont des virus, de préférence des phages tels que des phages filamenteux.

4. Procédé de sélection selon l'une quelconque des revendications 1 ou 2 caractérisé en ce que les microorganismes sont des phagémides.

5. Procédé de sélection selon l'une quelconque des revendications précédentes caractérisé en ce que le support solide est constitué par une colonne chromatographique sur laquelle est immobilisé de l'avidine, de la streptavidine et/ou un anticorps.

6. Procédé de sélection selon l'une quelconque des revendications précédentes caractérisé en ce que les molécules à activité enzymatique sont choisies parmi le groupe constitué par les enzymes, les abzymes, les peptides catalytiques ou un mélange d'entre-eux.

7. Procédé de sélection selon l'une quelconque des revendications précédentes caractérisé en ce que les microorganismes sont recueillis par détachement de leur support solide.

8. Procédé de sélection selon l'une quelconque des revendications précédentes, caractérisé en ce que les microorganismes recombinants sont recueillis par clivage du bras reliant l'inhibiteur covalent au support solide.

9. Procédé de sélection selon l'une quelconque des revendications 1 à 7, caractérisé en ce que les microorganismes recombinants sont recueillis en détachant le ligand du support solide avec une solution éluante, de préférence une solution à un pH acide.

10. Procédé de sélection selon l'une quelconque des revendications 1 à 7 caractérisé en ce que les microorganismes recombinants sont recueillis par clivage de la liaison entre l'inhibiteur covalent et la molécule à activité enzymatique.

11. Procédé de sélection selon l'une quelconque des revendications 1 à 7 caractérisé en ce que les microorganismes recombinants sont recueillis par clivage du lien peptidique entre la molécule à activité enzymatique et le microorganisme.

12. Procédé de sélection selon la revendication 11 caractérisé en ce que le clivage s'effectue par une protéase.

13. Procédé de sélection selon la revendication 11 caractérisé en ce que le clivage s'effectue par du bromure de cyanogène.

14. Procédé de sélection selon la revendication 11 caractérisé en ce que le clivage s'effectue par de l'hydroxylamine.

15. Procédé de sélection selon l'une quelconque des revendications 1 à 7 caractérisé en ce que les microorganismes recombinants sont détruits et que tout ou une partie de leur génome est recueilli, isolé et cloné.

16. Marqueur utilisé dans le procédé selon l'une quelconque des revendications précédentes 1 à 15 caractérisé en ce qu'il est constitué par un inhibiteur covalent du site actif d'une molécule à activité enzymatique, relié par un 5 bras à un ligand immobilisable sur un support solide.

17. Marqueur selon la revendication 16 caractérisé en ce que l'inhibiteur covalent est un inhibiteur irréversible.

18. Marqueur selon la revendication 16 ou 17 caractérisé en ce que le ligand est une biotine ou une iminobiotine.

19. Marqueur selon l'une quelconque des revendications 16 à 18, caractérisé en ce que le bras comporte une fonction clivable, de préférence une fonction disulfure.

20. Marqueur selon l'une quelconque des revendications précédentes 16 à 19, caractérisé en ce que l'inhibiteur covalent est un analogue de l'état de transition spécifique de la réaction enzymatique réalisée par la molécule à activité enzymatique.

21. Procédé de préparation du marqueur selon l'une quelconque des revendications 16 à 20 caractérisé en ce que l'on couple par un bras un inhibiteur covalent du site actif d'une molécule à activité enzymatique à un ligand immobilisable sur un support solide.

22. Support solide utilisé dans le procédé selon l'une quelconque des revendications 1 à 15 caractérisé en ce qu'il comprend un inhibiteur covalent du site actif d'une molécule à activité enzymatique, relié par un bras à un support solide.

23. Support solide selon la revendication 22 caractérisé en ce que l'inhibiteur covalent relié au support solide est constitué par le marqueur selon l'une quelconque des revendications 17 à 20.

24. Support solide selon la revendication 22 caractérisé en ce que le bras est relié au support solide par un lien ester ou comporte un diol vicinal.

25. Procédé de préparation d'un support solide selon l'une quelconque des revendications 22 à 24 caractérisé en ce que l'on relie par un bras un inhibiteur covalent du site actif d'une molécule à activité enzymatique à un support solide.

## Patentansprüche

1. Auswahlverfahren zur Auswahl von rekombinanten Mikroorganismen, die an ihrer Oberfläche mindestens ein Molekül mit enzymatischer Aktivität aufweisen, dadurch gekennzeichnet, daß die rekombinanten Mikroorganismen auf einem festen Träger immobilisiert werden durch einen kovalenten Inhibitor des aktiven Zentrums des Moleküls mit enzymatischer Aktivität, der über einen Arm mit dem festen Träger verbunden ist; daß die rekombinanten Mikroorganismen oder ihr ganzes Genom oder ein Teil ihres Genoms aufgefangen werden, und daß ihr ganzes Genom oder ein Teil ihres Genoms isoliert wird.

2. Auswahlverfahren gemäß Anspruch 1, dadurch gekennzeichnet, daß die rekombinanten Mikroorganismen vor ihrer Immobilisierung auf dem festen Träger markiert werden durch den kovalenten Inhibitor des aktiven Zentrums des Moleküls mit enzymatischer Aktivität, der über einen Arm mit einem auf dem festen Träger immobilisierbaren Liganden verbunden ist.

3. Auswahlverfahren gemäß irgendeinem der Ansprüche 1 oder 2, dadurch gekennzeichnet, daß die Mikroorganismen Viren, vorzugsweise Phagen, wie beispielsweise filamentöse Phagen sind.

4. Auswahlverfahren gemäß irgendeinem der Ansprüche 1 oder 2, dadurch gekennzeichnet, daß die Mikroorganismen Phagemiden sind.

5. Auswahlverfahren gemäß irgendeinem der vorhergehenden Ansprüche, dadurch gekennzeichnet, daß der feste Träger aus einer chromatographischen Säule besteht, auf der Avidin, Streptavidin und/oder ein Antikörper immobilisiert sind.

6. Auswahlverfahren gemäß irgendeinem der vorhergehenden Ansprüche, dadurch gekennzeichnet, daß die Moleküle mit enzymatischer Aktivität in der Gruppe gewählt werden, die von Enzymen, Abzymen, katalytischen Peptiden, oder einem Gemisch davon gebildet wird.

7. Auswahlverfahren gemäß irgendeinem der vorhergehenden Ansprüche, dadurch gekennzeichnet, daß die Mikroorganismen durch Ablösung von ihrem festen Träger aufgefangen werden.

8. Auswahlverfahren gemäß irgendeinem der vorhergehenden Ansprüche, dadurch gekennzeichnet, daß die rekombinanten Mikroorganismen durch Aufspaltung des Arms, der den kovalenten Inhibitor mit dem festen Träger verbindet, aufgefangen werden.

9. Auswahlverfahren gemäß irgendeinem der Ansprüche 1 bis 7, dadurch gekennzeichnet, daß zum Auffangen der rekombinanten Mikroorganismen der Ligand mit einer Eluierungslösung, vorzugsweise einer Lösung mit einem sauren pH, von dem festen Träger abgelöst wird.

10. Auswahlverfahren gemäß irgendeinem der Ansprüche 1 bis 7, dadurch gekennzeichnet, daß die rekombinanten Mikroorganismen durch Aufspaltung der Bindung zwischen dem kovalenten Inhibitor und dem Molekül mit enzymatischer Aktivität aufgefangen werden.

11. Auswahlverfahren gemäß irgendeinem der Ansprüche 1 bis 7, dadurch gekennzeichnet, daß die rekombinanten Mikroorganismen durch Aufspaltung der peptidbindung zwischen dem Molekül mit enzymatischer Aktivität und dem Mikroorganismus aufgefangen werden.

12. Auswahlverfahren gemäß Anspruch 11, dadurch gekennzeichnet, daß die Aufspaltung durch eine Protease erfolgt.

13. Auswahlverfahren gemäß Anspruch 11, dadurch gekennzeichnet, daß die Aufspaltung durch Cyanbromid erfolgt.

14. Auswahlverfahren gemäß Anspruch 11, dadurch gekennzeichnet, daß die Aufspaltung durch Hydroxylamin erfolgt.

15. Auswahlverfahren gemäß irgendeinem der Ansprüche 1 bis 7, dadurch gekennzeichnet, daß die rekombinanten Mikroorganismen zerstört werden, und daß ihr ganzes Genom oder ein Teil ihres Genoms aufgefangen, isoliert und kloniert wird.

16. Marker, der bei dem Verfahren gemäß irgendeinem der vorhergehenden Ansprüche 1 bis 15 verwendet wird, dadurch gekennzeichnet, daß er aus einem kovalenten Inhibitor des aktiven Zentrums eines Moleküls mit enzymatischer Aktivität besteht, der über einen Arm mit einem auf einem festen Träger immobilisierbaren Liganden verbunden ist.

17. Marker gemäß Anspruch 16, dadurch gekennzeichnet, daß der kovalente Inhibitor ein irreversibler Inhibitor ist.

18. Marker gemäß Anspruch 16 oder 17, dadurch gekennzeichnet, daß der Ligand ein Biotin oder ein Iminobiotin ist.

19. Marker gemäß irgendeinem der Ansprüche 16 bis 18, dadurch gekennzeichnet, daß der Arm eine aufspaltbare Gruppe, vorzugsweise eine Disulfidgruppe umfaßt.

20. Marker gemäß irgendeinem der vorhergehenden Ansprüche 16 bis 19, dadurch gekennzeichnet, daß der kovalente Inhibitor dem spezifischen Übergangszustand der durch das Molekül mit enzymatischer Aktivität verwirklichten enzymatischen Reaktion entspricht.

21. Verfahren zur Herstellung des Markers gemäß irgendeinem der Ansprüche 16 bis 20, dadurch gekennzeichnet, daß ein kovalenter Inhibitor des aktiven Zentrums eines Moleküls mit enzymatischer Aktivität über einen Arm mit einem auf einem festen Träger immobilisierbaren Liganden verbunden wird.

22. Fester Träger, der bei dem Verfahren gemäß irgendeinem der Ansprüche 1 bis 15 verwendet wird, dadurch gekennzeichnet, daß er einen kovalenten Inhibitor des aktiven Zentrums eines Moleküls mit enzymatischer Aktivität aufweist, der über einen Arm mit einem festen Träger verbunden ist.

23. Fester Träger gemäß Anspruch 22, dadurch gekennzeichnet, daß der mit dem festen Träger verbundene, kovalente Inhibitor aus dem Marker gemäß irgendeinem der Ansprüche 17 bis 20 besteht.

24. Fester Träger gemäß Anspruch 22, dadurch gekennzeichnet, daß der Arm über eine Esterbindung mit dem festen Träger verbunden ist, oder ein vicinales Diol umfaßt.

25. Verfahren zur Herstellung eines festen Trägers gemäß irgendeinem der Ansprüche 22 bis 24, dadurch gekennzeichnet, daß ein kovalenter Inhibitor des aktiven Zentrums eines Moleküls mit enzymatischer Aktivität über einen Arm mit einem festen Träger verbunden wird.

## Claims

1. Method for selecting recombinant microorganisms containing at their surface at least one molecule having enzymatic activity, characterized in that the recombinant microorganisms are immobilized on a solid support by a covalent inhibitor of the active site of the molecule having enzymatic activity, connected to the solid support by an arm; in that the recombinant microorganisms or all or part of their genome are recovered and in that all or part of their genome is isolated.

2. Method of selection according to claim 1, characterized in that prior to their immobilization on the solid support, the recombinant microorganisms are labeled with the covalent inhibitor of the active site of the molecule having enzymatic activity, connected by an arm to a ligand which can be immobilized on the solid support.

3. Method of selection according to any one of claims 1 or 2, characterized in that the microorganisms are viruses, preferably phages such as filamentous phages.

4. Method of selection according to any one of claims 1 or 2, characterized in that the microorganisms are phagemids.

5. Method of selection according to any one of the preceding claims, characterized in that the solid support consists of a chromatographic column on which avidin, streptavidin and/or an antibody is immobilized.

6. Method of selection according to any one of the preceding claims, characterized in that the molecules having enzymatic activity are chosen from the group consisting of enzymes, abzymes, catalytic peptides or a mixture thereof.

7. Method of selection according to any one of the preceding claims, characterized in that the microorganisms are recovered by detachment from their solid support.

8. Method of selection according to any one of the preceding claims, characterized in that the recombinant microorganisms are recovered by cleavage of the arm connecting the covalent inhibitor to the solid support.

9. Method of selection according to any one of claims 1 to 7, characterized in that the recombinant microorganisms are recovered by detaching the ligand from the solid support with an eluting solution, preferably a solution at an acidic pH.

10. Method of selection according to any one of claims 1 to 7, characterized in that the recombinant microorganisms are recovered by cleavage of the bond between the covalent inhibitor and the molecule having enzymatic activity.

11. Method of selection according to any one of claims 1 to 7, characterized in that the recombinant microorganisms are recovered by cleavage of the peptide linkage between the molecule having enzymatic activity and the microorganism.

12. Method of selection according to claim 11, characterized in that the cleavage is performed with a protease.

13. Method of selection according to claim 11, characterized in that the cleavage is performed with cyanogen bromide.

14. Method of selection according to claim 11, characterized in that the cleavage is performed with hydroxylamine.

15. Method of selection according to any one of claims 1 to 7, characterized in that the recombinant microorganisms are destroyed and that all or part of their genome is recovered, isolated and cloned.

16. Marker used in the process according to any of the preceding claims characterized in that it consists of a covalent inhibitor of the active site of a molecule having enzymatic activity, connected by an arm to a ligand which can be immobilized on a solid support.

17. Marker according to claim 16, characterized in that the covalent inhibitor is an irreversible inhibitor.

18. Marker according to claim 16 or 17, characterized in that the ligand is a biotin or an iminobiotin.

19. Marker according to any one of claims 16 to 18, characterized in that the arm contains a cleavable functional group, preferably a disulfide functional group.

20. Marker according to any one of the preceding claims 16 to 19, characterized in that the covalent inhibitor is an analog of the specific transition state of the enzymatic reaction performed by the molecule having enzymatic activity.

21. Method for preparing the marker according to any one of claims 16 to 20, characterized in that a covalent inhibitor of the active site of a molecule with enzymatic activity is coupled by an arm to a ligand which can be immobilized on a solid support.

22. Solid support used in the process according to any of the preceding claims, characterized in that it comprises a covalent inhibitor of the active site of a molecule having enzymatic activity, connected to a solid support by an arm.

23. Solid support according to claim 22, characterized in that the covalent inhibitor connected to the solid support consists of the marker according to any one of claims 17 to 20.

24. Solid support according to claim 22, characterized in that the arm is connected to the solid support by an ester linkage or contains a vicinal diol.

25. Method for preparing a solid support according to any one of claims 22 to 24, characterized in that a covalent inhibitor of the active site of a molecule having enzymatic activity is connected by an arm to a solid support.
